# EUROPÄISCHE PATENTSCHRIFT

(11) **EP 1 486 176 B1**
(45) Veröffentlichungstag und Bekanntmachung des Hinweises auf die Patenterteilung: **02.04.2008**
(21) Anmeldenummer: 04010254.3
(22) Anmeldetag: 30.04.2004
(51) Int. Cl.: A61B 17/92, A61B 17/72, A61B 17/17

(54) **Kombination aus intramedullärem Nagel und Ziel- und/oder Einschlaginstrument**
Combination of an intramedullary pin and a targering device or/and a percussion device
Combinaison d'un clou intramédullaire et d'un instrument de visée ou/et de percussion

(30) Priorität: 11.06.2003 DE 20309058 U
(43) Veröffentlichungstag der Anmeldung: 15.12.2004
(73) Patentinhaber: Stryker Trauma GmbH, 24232 Schönkirchen (DE)
(72) Erfinder: Bigdeli-Issazadeh, Sabine, 24242 Felde (DE); Cremer, Axel, 23795 Fahrenkrog (DE)
(74) Vertreter: Kopf, Korbinian Paul

(56) Entgegenhaltungen:
- EP-A- 0 441 256
- EP-A- 0 888 751
- US-A- 5 499 986
- US-A- 6 010 508

## Beschreibung

Die Erfindung bezieht sich auf eine Kombination aus intramedulärem Nagel unter einem Ziel- und/oder Einschlaginstrument nach dem Obergriff des Anspruchs 1.

Intrameduläre Nägel werden zumeist mit einem sogenannten Einschlaginstrument in den Knochenkanal eingetrieben. Das Einschlaginstrument wird über eine geeignete Verbindung mit dem Nagel verbunden, bevor mit Hilfe eines Hammers oder dergleichen der Nagel vorgetrieben wird. Bei sogenannten Verriegelungsnägeln, die mit Querbohrungen versehen sind, zur Aufnahme von Knochen- oder Verriegelungsschrauben, dient das Zielgerät zugleich als Einschlaginstrument. Das Zielgerät ist über einen Bügel mit dem zugewandten Ende des Knochennagels verbunden, und ein parallel zum Knochen verlaufender Zielabschnitt dient zum Auffinden der ansonsten unsichtbaren Querbohrungen des Nagels im Knochen. Aus diesem Grunde ist erforderlich, Nagel und Zielgerät in vorgegebener Drehlage zueinander auszurichten. Zu diesem Zweck ist bekannt geworden, am Anschlußende des Zielgeräts einen achsparallelen Vorsprung vorzusehen, der in eine achsparallele Ausnehmung des Anschlußendes des Nagels eingreift. Die Verbindung des Anschlußendes des Zielgerätes mit dem Nagel erfolgt zumeist mit Hilfe einer Schraube oder auch Schraubhülse, wie aus U.S. 5176681 bekannt geworden ist. Die Schraubhülse dienst dazu, einen Durchgang zum Nagelinnern freizulassen, damit es möglich ist, eine Verriegelungsschraube in den Nagel einzubringen oder zu betätigen, während das Zielgerät mit dem Nagel verbunden ist. Die Verriegelungsschraube dient dazu, eine Schenkelhalsschraube, die durch eine schräge Querbohrung des Nagels hindurchgeführt ist, zu verriegeln, insbesondere in Drehrichtung, eine axiale Bewegung aber zuzulassen.

Das bekannte System erfordert das Ansetzen des Nagels am Zielgerät von Hand und das gleichzeitige Verschrauben der Teile miteinander, was für eine einzige Person verhältnismässig umständlich ist.

Aus EP 0 888 751 A2 ist eine Vorrichtung bekannt zum lösbaren Befestigen eines Operationsgegenstandes mit einem chirurgischen Element oder Werkzeug, wobei eine Verdrehung der Elemente durch eine Abplattung an einem entfernten Ende eines Elementes vorgeschlagen wird, sowie eine einseitige Kopplung an einer Hinterschneidung eines Vorsprunges.

Der Erfindung liegt daher die Aufgabe zugrunde, die Handhabung der Verbindung zwischen dem Nagel und dem Ziel- und/oder Einschlaginstrument zu vereinfachen.

Diese Aufgabe wird durch die Merkmale des Anspruchs 1 gelöst. Vorteilhafte Ausgestaltungen werden durch die abhängigen Ansprüche verkörpert.

Bei der Erfindung ist ein Schnellverschlusselement am Anschlussende des Ziel und/oder Einschlaginstruments drehbar gelagert. Es greift in das hohle Anschlussende des Nagels ein und weist ein erstes Kupplungsmittel auf. Das Anschlussende des Nagels weist in seinem Inneren ein zweites Kupplungsmittel auf und die Kupplungsmittel sind so ausgebildet, dass in einer ersten Drehposition das Schnellverschlusselement, in der das Schnellverschlusselement eine relative Drehposition zum achsparallelen Vorsprung aufweist, in das Anschlussende des Nagels einführbar ist und die Kupplungsmittel in einer zweiten Drehposition des Schnellverschlusselements zusammenwirken, um den Nagel axial fest am Ziel- und Einschlaginstrument zu halten.

Bei dem Zusammenbringen von Nagel und Ziel- und/oder Einschlaginstrument wird der Nagel zum einen gegenüber dem Instrument in der richtigen Drehlage positioniert, damit der achsparallele Vorsprung in die achsparallele Ausnehmung des Nagels eingreifen kann. Zugleich wird das Schnellverschlusselement in eine Position gedreht, in der es in das offene Ende des Nagels eingeführt werden kann. Anschliessend wird das Schnellverschlusselement in seine zweite Position verdreht, wobei die Kupplungsmittel miteinander in Eingriff treten, um eine axiale Festlegung des Nagels am Ziel- und Einschlaginstrument herbeizuführen. Eine Festlegung in Drehrichtung ist bereits durch das Eingreifen des achsparallelen Vorsprungs des Anschlußendes des Instruments in die achsparallele Ausnehmung des Nagels erfolgt.

Die erfindungsgemäße Ausführung hat den Vorteil, daß das Anbringen eines Nagels Ziel- und Einschlaginstrument und auch das Lösen erheblich vereinfacht wird. Erforderlich ist, daß der Nagel am Anschlußende eine entsprechende Ausbildung erhält, wodurch ein Kupplungsmittel geschaffen ist, das mit dem Kupplungsmittel des Schnellverschlußelements zusammenwirkt.

Die Handhabung bei der Erfindung wird noch verbessert, wenn das Schnellverschlußelement in der ersten Drehposition von einer Feder in Richtung zweite Drehposition vorgespannt wird. Die Kupplungsmittel sind so ausgeführt, daß sie automatisch zusammenwirken, wenn das Schnellverschlußelement eine vorgegebene Strecke axial in das Anschlußende des Nagels eingesetzt worden ist.

Eine Möglichkeit für die Ausbildung des Kupplungsmittels im Nagel besteht darin, daß ein Gewindeabschnitt vorgesehen ist. Ferner wird mindestens eine achsparallele Nut im Nagel geformt, mit der ein radialer Vorsprung des Schnellverschlußelements zusammenwirkt. Der Vorsprung wird in die achsparallele Nut des Nagels um eine vorgegebene Strecke eingeführt, und anschließend bei Drehung des Schnellverschlußelements mit einem Gangabschnitt bzw. einer Nut des Gewindeabschnitts zusammengebracht. Es versteht sich, daß die Steigung des Gewindeabschnitts im Nagel äußerst gering ist, da das Schnellverschlußelement vorzugsweise axial fest im Anschlußende des Ziel- und/oder Einschlaginstruments gelagert ist. Vorzugsweise sind drei im Umfangsabstand von 120 ° angeordnete radiale Vorsprünge am Schnellverschlußelement vorgesehen, die axial um die Gewindesteigung beabstandet sind. Der Gewindeabschnitt ist dann so ausgebildet, daß jeweils ein Vorsprung mit einem Gangabschnitt des Gewindeabschnitts zusammenwirkt.

Nach einer Ausgestaltung der Erfindung ist das Schnellverschlußelement eine Hülse, die axial fest, jedoch begrenzt drehbar in einer Bohrung des Anschlußendes des Ziel- und Einschlaginstruments gelagert ist. Die Hülse weist einen radialen Betätigungsstift auf, der sich durch einen radialen Schlitz des Anschlußendes nach außen erstreckt. Es ist zwar denkbar, statt einer Hülse auch einen zylindrischen Körper als Schnellverschlußelement zu verwenden. Die Hülse hat den Vorteil, daß über die Hülse Zugang zum Nagelinnern erhalten wird, etwa zur Betätigung eines Verriegelungsstiftes im Nagel.

Es wurde bereits erwähnt, daß das Schnellverschlußelement in Drehrichtung vorgespannt sein kann. Nach einer Ausgestaltung der Erfindung ist die Feder zum Vorpannen des Schnellverschlußelements eine Schraubenfeder, welche zum einen mit der Hülse und zum anderen mit der Bohrung des Anschlußendes des Instruments zusammenwirkt.

Um eine sichere Anlage des Ziel- und Einschlaginstruments am Nagel zu gewährleisten, ist es nach einer weiteren Ausgestaltung der Erfindung vorteilhaft, wenn das Anschlußende des Ziel und Einschlaginstruments einen hülsenförmigen Endabschnitt aufweist, der annähernd passend in einen entsprechenden Bohrungsabschnitt des Anschlußendes des Nagels eingreift.

Die Erfindung wird nachfolgend anhand eines in Zeichnungen dargestellten Ausführungsbeispiels näher erläutert.
Figur 1 zeigt perspektivisch das Anschlußende eines Ziel- und Einschlaginstruments.
Figur 2 zeigt vergrößert die Seitenansicht des Instruments nach Figur 1.
Figur 3 zeigt einen Schnitt durch die Darstellung nach Figur 2 entlang der Linie 3-3.
Figur 4 zeigt einen Schnitt durch die Darstellung nach Figur 2 entlang der Linie 4-4.
Figur 5 zeigt die Seitenansicht des Instruments gemäß Pfeil 5 nach Figur 3.
Figur 6 zeigt vergrößert den Teil der Darstellung nach Figur 5 im Kreis 6.
Figur 7 zeigt die Untersicht unter das Instrument gemäß Figur 3 oder Figur 5.
Figur 8 zeigt eine ähnliche Darstellung wie Figur 2, jedoch mit aufgenommenen Schnellverschlußelement.
Figur 9 zeigt einen Schnitt durch die Darstellung nach Figur 8 entlang der Linie 9-9.
Figur 10 zeigt eine Draufsicht auf die Darstellung nach den Figuren 8 oder 9.
Figur 11 zeigt einen Schnitt durch die Darstellung nach Figur 10 entlang der Linie 11-11.
Figur 12 zeigt das Anschlußende eines nicht weiter dargestellten Verriegelungsnagels.
Figur 13 zeigt den Schnitt durch den Nagel nach Figur 12 entlang der Linie 13-13.
Figur 14 zeigt die Endansicht des Nagels nach Figur 12 in Richtung Pfeil 14.
Figur 15 zeigt vergrößert den Abschnitt 15 nach Figur 13.

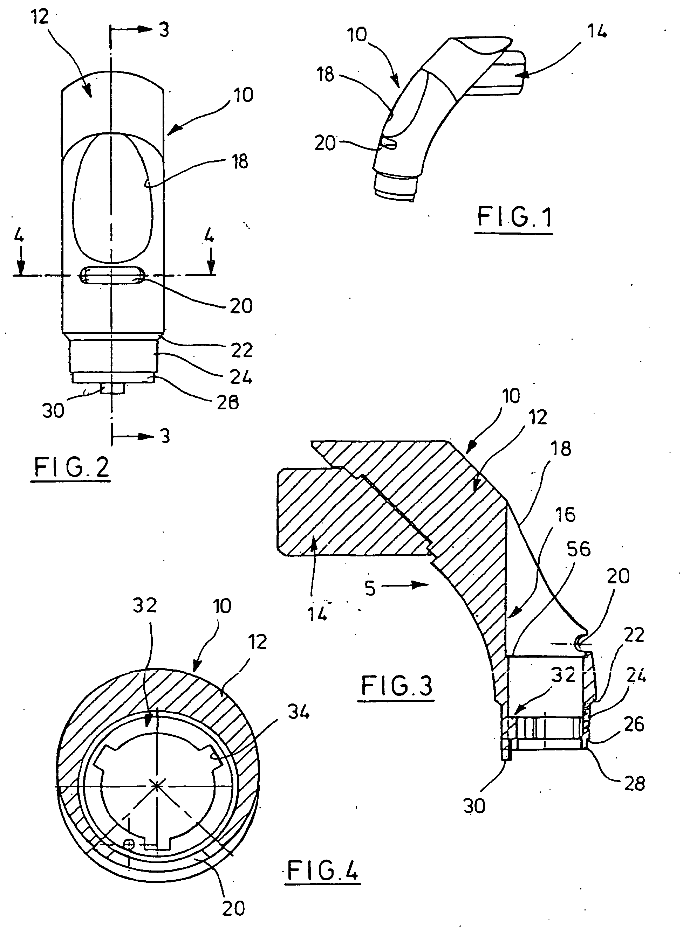

In den Figuren 1 bis 3 ist ein Anschlußende 10 eines nicht im einzelnen dargestellten Einschlag- und Zielinstruments zu erkennen. Es weist einen ersten Teil 12 auf und einen zweiten Teil 14, die miteinander durch Schweißung verbunden sind. Der Teil 14 dient zur Verbindung mit dem übrigen Abschnitt des Zielgeräts, der nicht gezeigt ist und für den Gegenstand der Beschreibung keine Bedeutung hat.

Das bogenförmig gekrümmte, im Querschnitt runde Teil 12 weist eine gerade Durchbohrung 16 auf, die nach oben in eine schräge Öffnung 18 mündet. Kurz unterhalb der Öffnung 18 ist ein radialer Schlitz 20 im Teil 12 geformt; unterhalb des Schlitzes 12 befindet sich außen eine konische Stufe 22, unter der sich ein zylindrischer Abschnitt 24 anschließt. Der zylindrische Abschnitt 24 geht über eine weitere radiale Stufe 26 in einen zylindrischen Abschnitt 28 über, der wiederum einen kleineren Außendurchmesser hat. An diesen schließt sich ein achsparalleler Vorsprung 30 an. Im Inneren der Bohrung 16 befindet sich in Höhe des zylindrischen Abschnitts 24 ein nach innen weisender radialer Bund 32, der, wie in Figur 4 zu erkennen, drei im 120° Abstand angeordnete radiale Ausnehmungen 34 besitzt.

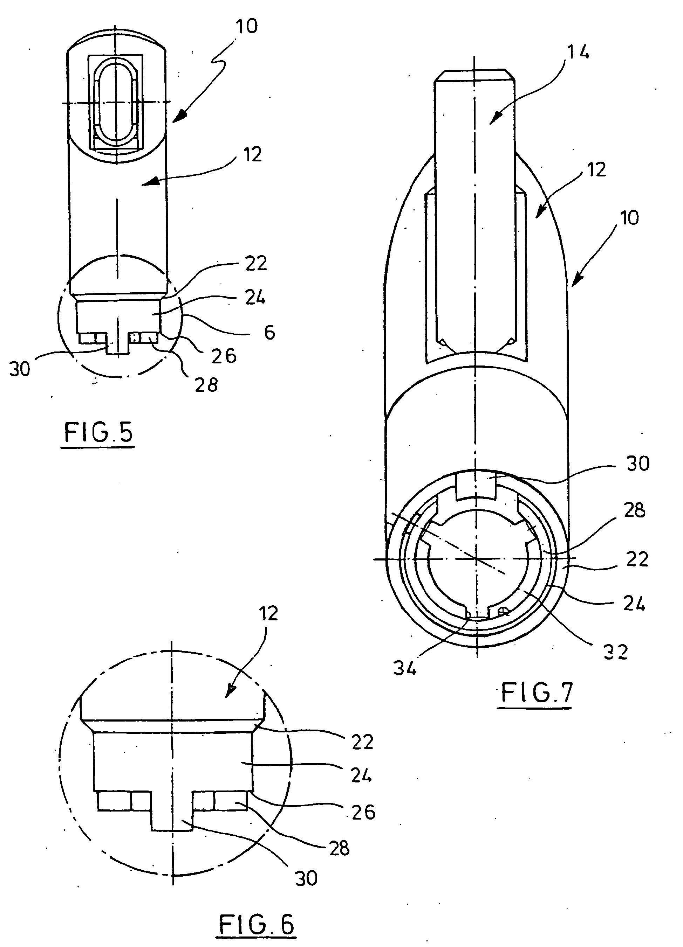

Aus den Figuren 5 bis 7 ist zu erkennen, daß der Außendurchmesser des achsparallelen Vorsprungs 30 dem des zylindrischen Abschnitts 24 entspricht. Der Abschnitt 28 endet auf beiden Seiten des Vorsprungs 30 im Abstand zu diesem, wie auch insbesondere in Figur 7 zu erkennen.

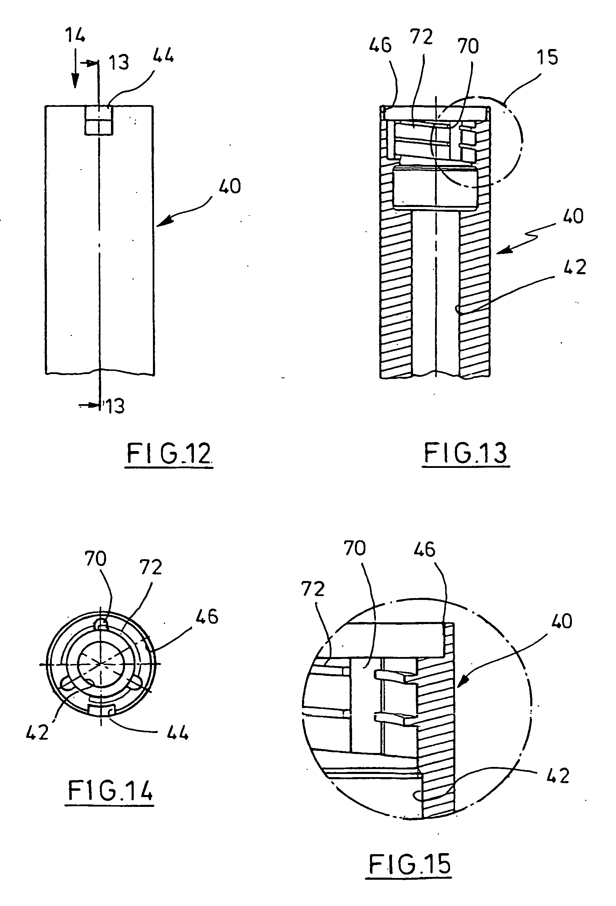

In den Figuren 12 bis 15 ist ein Nagelabschnitt 40 zu erkennen. Er ist Teil eines zylindrischen Nagelschaftes, der eine axiale Bohrung 42 aufweist. Wie aus Figur 12 zu erkennen, weist der Nagelabschnitt 40 am oberen Ende eine achsparallele Ausnehmung 44 auf. Die Bohrung 42 ist nach oben erweitert, und am oberen Ende ist ein Bohrungsabschnitt 46 vorgesehen. Der Innendurchmesser des Bohrungsabschnitts 46 entspricht dem Außendurchmesser des zylindrischen Abschnitts 28 des Anschlußendes des Instruments 10. Das Anschlußende kann daher mit dem Abschnitt 28 in den Bohrungsabschnitt 46 eingesetzt werden, wobei der achsparallele Vorsprung 30 passend in die Ausnehmung 44 eingreift. Der Außendurchmesser des Nagelabschnitts 40 entspricht dem Außendurchmesser des Abschnitts 24 des Anschlußendes des Instruments 10.

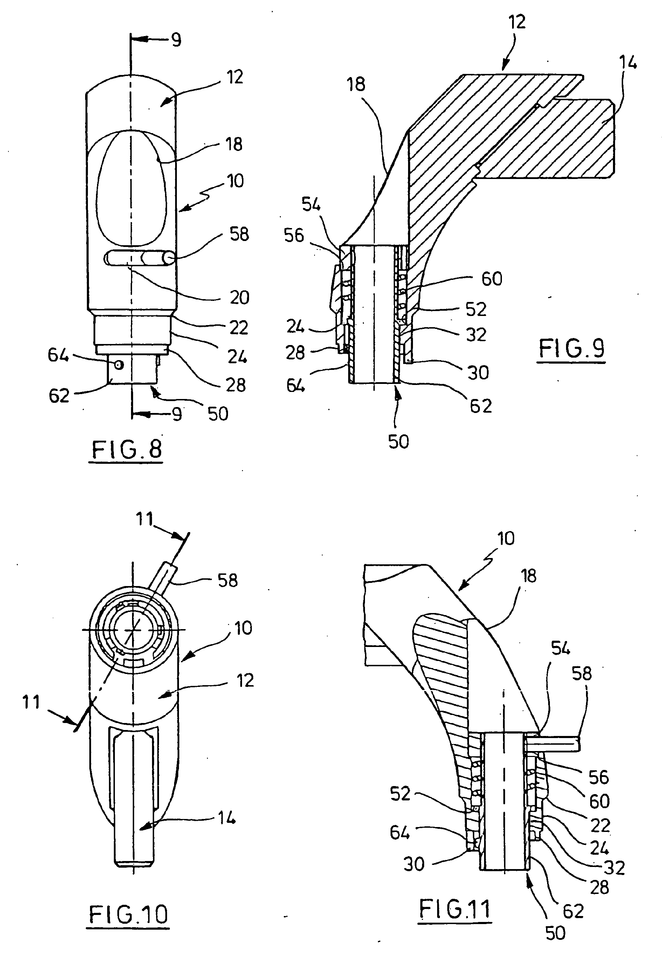

In den Figuren 8 bis 11 ist zu erkennen, daß in das Anschlußende 10 des Instruments ein Schnellverschlußelement eingesetzt ist. Das Schnellverschlußelement ist eine Hülse 50, die in die Bohrung 16 des Teils 12 eingesetzt ist. Die Hülse 50 weist einen radialen Bund 52 auf, der mit der Schulter zusammenwirkt, welche von dem radialen Bund 32 gebildet ist. Im axialen Abstand zum radialen Bund 52 weist die Hülse 50 am linken Ende einen weiteren radialen Bund 54 auf, der gegen einen Absatz 56 zur Anlage gelangen kann. In eine Bohrung des Bundes 54 ist radial ein Betätigungsstift 58 eingepreßt, der sich durch den radialen Schlitz 20 hindurcherstreckt. Zwischen der Hülse 50 und dem Bohrungsabschnitt zwischen dem Absatz 56 und dem Bund 32 ist ein ringförmiger Zwischenraum gebildet, in dem eine Schraubenfeder 60 angeordnet ist. Das eine Ende der Schraubenfeder ist fest mit dem Teil 12 verbunden und das andere Ende ist mit der Hülse 50 verbunden. Eine Drehung der Hülse 50 führt daher zu einer Vorspannung der Hülse 50 durch die Feder 60.

In der eingebauten Position steht die Hülse 50 nach unten über, wie sich aus den Figuren 8, 9 und 11 ergibt. Wie aus diesen Figuren ferner erkennbar, ist der überstehende Hülsenabschnitt 62 mit kurzen radialen Zapfen 64 versehen. Insgesamt sind drei im Umfangsabstand von 120° angeordnete Zapfen 64 vorgesehen, die außerdem einen axialen Abstand aufweisen. Auf diesen Abstand wird weiter unten eingegangen.

Aus den Figuren 13 bis 15 kann man erkennen, daß unterhalb des Bohrungsabschnitts 46 im Nagelabschnitt 40 drei achsparallele Nuten in die Wandung der Bohrung eingeformt sind. Sie sind mit 70 bezeichnet. Die Nuten 70 unterbrechen einen Gewindeabschnitt 72, der unterhalb des Bohrungsabschnitts 46 eingeformt ist. Die Steigung dieses Gewindes ist außerordentlich flach.

Wird, wie oben bereits beschrieben, das Abschlußende des Instruments 10 in den Nagelabschnitt 40 eingesetzt, in dem der zylindrische Abschnitt 28 in den Bohrungsabschnitt 46 eingreift und der Vorsprung 30 in die Ausnehmung 44, dann kann dies nur bewerkstelligt werden, wenn außerdem die Hülse 50 eine Drehlage hat, daß jeweils ein Zapfen 64 in eine Nut 70 eingeführt werden kann. Um diese Drehlage zu erhalten, muß die Hülse 50 entsprechend verdreht werden, und zwar mit Hilfe des Betätigungsstiftes 58. Bei dieser Verdrehung wird die Schraubenfeder 60 gespannt. Wird in der beschriebenen Ausrichtung der Teile nunmehr der Nagel vollständig an den Teil 12 des Anschlußendes 10 des Instruments eingeschoben, werden die Zapfen 64 zu jeweils einem Gewindegang des Gewindeabschnitts 72 bzw. zu einer Gewindenut ausgerichtet. Der axiale Abstand der Zapfen 64 ist nämlich derart, daß er der Steigung des Gewindeabschnitts 72 entspricht. Wird nun bei der beschriebenen Ausrichtung der Teile der Betätigungsstift 58 losgelassen, wird die Hülse 50 von der Feder 60 in die entspannte Lage zurückzudrehen versucht, wodurch die Zapfen 64 in jeweils einen Nutabschnitt des Gewindeabschnitts 72 eingreifen und dadurch eine Verriegelung mit dem Nagelabschnitt 40 erstellen. Auf diese Weise ist der Nagelabschnitt 40 sowohl in Drehrichtung als auch axialer Richtung fest an Anschlußende 10 festgelegt.

Eine alternative Anbringung des Nagelabschnitts 40 am Instrument 10 geschieht wie folgt. Das Nagelende 40 wird über den vorstehenden Hülsenabschnitt 62 geschoben, wobei die Zapfen 64 in die Nuten 70 eingreifen. Anschließend wird der Nagelabschnitt 40 gegenüber dem Instrument 10 soweit verdreht, bis der Vorsprung 30 mit der Ausnehmung 44 des Nagelabschnitts ausgerichtet ist. Nach dieser Ausrichtung wird der Nagel weiter gegen das Instrument 10 vorgeschoben, so daß der Vorsprung 30 vollständig in die Ausnehmung 40 eingreift. Sobald dies beendet ist, gelangen die Zapfen 64 in Ausrichtung mit den Nutabschnitten des Gewindeabschnittes 72 und bewegen sich automatisch in diese hinein, da bei der beschriebenen Drehung die Schraubenfeder 60 vorgespannt wurde und versucht, die Hülse 50 in unbekehrter Richtung zurückzudrehen.

Aus der Erläuterung wird deutlich, daß in den beschriebenen Figuren ein Schnellverschlußsystem zu erkennen ist, mit dem ein Nagel, beispielsweise ein Verriegelungsnagel, rasch ohne Zuhilfenahme von Instrumenten oder einer Schraube oder dergleichen mit einem Ziel- und/oder Einschlaginstrument verbunden werden kann.

## Patentansprüche

1. Kombination aus intramedulärem Nagel und Ziel- und/oder Einschlaginstrument, wobei ein Anschlussende (10) des Ziel- und/oder Einschlaginstruments einen achsparallelen Vorsprung (30) hat, der mit einer achsparallelen Ausnehmung (44) am hohlen Anschlussende (10) des Nagels zusammenwirkt, so daß der Nagel durch dieses Zusammen wirken unter axialer Anlage am Anschlussende des Ziel- und Einschlaginstruments in vorgegebener Drehlage am Ziel- und/oder Einschlaginstrument fest gelegt wird und Mittel vorgesehen sind, den Nagel mit dem Ziel- und/oder Einschlaginstrument axial fest lösbar zu kuppeln, wobei am Anschlussende (10) des Ziel- und/oder Einschlaginstruments ein Schnellverschlusselement drehbar gelagert ist, das in das Anschlussende des Nagels (40) eingreift und erste Kupplungsmittel aufweist und das Anschlussende des Nagels (40) im Innern zweite Kupplungsmittel aufweist und die Kupplungsmittel so ausgebildet sind, dass in einer ersten Drehposition des Schnellverschlusselements, bei der das Schnellverschlusselement eine relative Drehposition zum achsparallelen Vorsprung aufweist, in das Anschlussende des Nagels einführbar ist und die Kupplungsmittel in einer zweiten Drehposition des Schnellverschlusselements so zusammenwirken, dass der Nagel axial fest am Ziel- und/oder Einschlaginstrument gehalten ist.

2. Kombination nach Anspruch 1, **dadurch gekennzeichnet, dass** das Schnellverschlusselement in der ersten Drehposition von einer Feder (60) in Richtung zweiter Drehposition vorgespannt ist und die Kupplungsmittel so ausgeführt sind, dass sie automatisch zusammenwirken, wenn das Schnellverschlusselement eine vorgegebene Strecke axial in das Anschlussende des Nagels (40) eingeführt worden ist.

3. Kombination nach Anspruch 1 oder 2, **dadurch gekennzeichnet, dass** das Anschlussende des Nagels (40) im Inneren einen Gewindeabschnitt (72) aufweist und mindestens eine achsparallele Nut (70), das Schnellverschlusselement mindestens einen radialen Vorsprung (64) aufweist, der in die achsparallele Nut (70) einführbar ist und bei Drehung des Schnellverschlusselements von der ersten in die zweite Drehposition mit einem Gangabschnitts des Gewindeabschnitts (72) zusammenwirkt.

4. Kombination nach Anspruch 3, **dadurch gekennzeichnet, dass** drei radiale Vorsprünge (64) im 120° Abstand und um eine Gewindesteigung eines Gewindeabschnittes (72) axial versetzt angeordnet sind und das Innere des Nagels (40 3) im 120° versetzt angeordnete achsparallele Nuten aufweist, wobei der Gewindeabschnitt (72) so ausgebildet ist, dass jeweils ein Vorsprung (64) mit einem Gangabschnitt des Gewindeabschnitts (72) zusammenwirkt.

5. Kombination nach Anspruch 4, **dadurch gekennzeichnet, dass** das Schnellverschlusselement eine Hülse (50) aufweist, die axial fest, jedoch begrenzt drehbar in einer Bohrung (16) des Anschlussendes (10) des Ziel- und/oder Einschlagelements gelagert ist und die Hülse (50) mit einem radialen Betätigungsstift (58) versehen ist, der über einen radialen Schlitz (20) des Anschlussendes (12) nach Aussen ragt.

6. Kombination nach Anspruch 5 in verbindung mit Anspruch 2, **dadurch gekennzeichnet, dass** die Feder (60) in einem Zwischenraum zwischen Hülse (50) und Wandung der Bohrung (16) im Anschlussende (10) des Ziel- und/oder Einschlagelements angeordnet ist, deren eines Ende am Anschlussende und deren anderes Ende an der Hülse (50) festgelegt ist.

7. Kombination nach Anspruch 5 und 6, **dadurch gekennzeichnet, dass** die Hülse (50) einen ersten radialen Bund (52) aufweist, der gegen eine radiale Widerlagerfläche (32) in der Bohrung (16) des Anschlussendes (10) anliegt und die Hülse (50) mittels des Betätigungsstiftes (58) in der entgegengesetzten Richtung axial festgelegt ist, wobei die Feder (60) unter axialer Vorspannung gegen den Bund (52) der Hülse (50) und gegen den Betätigungsstift oder einem weiteren den Betätigungsstift (58) tragenden radialen Bund anliegt.

8. Kombination nach einem der Ansprüche 1 bis 7, **dadurch gekennzeichnet, dass** das Anschlussende (10) des Ziel- und/oder Einschlaginstruments einen hülsenförmigen Endabschnitt (28) aufweist, der annähernd passend in einen endseitigen Bohrungsabschnitt (46) des Anschlussendes des Nagels (40) eingreift.

9. Kombination nach Anspruch 8, **dadurch gekennzeichnet, dass** der achsparallele Vorsprung (30) des Anschlussendes (12) des Ziel- und/oder Einschlaginstruments über das freie Ende des hülsenförmigen Endabschnitts (28) axial übersteht.

## Claims

1. Combination of an intramedullary nail and a targeting and/or nailing instrument, wherein a connecting end (10) of the targeting and/or nailing instrument comprises an axially parallel projection (30), which cooperates with an axially parallel recess (44) on the hollow connection end (10) of the nail so that, due to this cooperation, the nail is placed under axial engagement on the connection end of the targeting and nailing instrument in a predefined rotational position on the targeting and/or nailing instrument and means are provided for coupling the nail with the targeting and or nailing instrument in an axially fixed and detachable manner, wherein rotationally mounted on the connection end (10) of the targeting and/or nailing instrument there is a quick-fastener element, which engages in the connection end of the nail (40) and comprises first coupling means and the connection end of the nail (40) comprises interior second coupling means and the coupling means are embodied so that in a first rotational position of the quick-fastener element, in which the quick-fastener element comprises a relative rotational position relative to the axially parallel projection [and] can be introduced into the connection end of the nail and the coupling means cooperate in a second rotational position of the quick-fastener element so that the nail is held in an axially fixed manner on the targeting and/or nailing instrument.

2. Combination according to claim 1, **characterised in that** the quick-fastener element is prestressed in the first rotational position by a spring (60) in the direction of the second rotational position and the coupling means are embodied so that they automatically cooperate when the quick-fastener element has been introduced axially into the connection end of the nail (40) by a predefined distance.

3. Combination according to claim 1 or claim 2, **characterised in that** the connection end of the nail (40) comprises an interior threaded section (72) and at least one axially parallel groove (70), the quick-fastener element comprises at least one radial projection (64), which can be introduced into the axially parallel groove (70) and on the rotation of the quick-fastener element from the first into the second rotational position cooperates with a pitch section of the threaded section (72).

4. Combination according to claim 3, **characterised in that** three radial projections (64) are arranged with a 120° spacing axially offset around a thread pitch of a threaded section (72) and the interior of the nail (40 3) comprises parallel grooves arranged axially offset at 120°, wherein the threaded section (72) is embodied so that each projection (64) cooperates with a pitch section of the threaded section (72).

5. Combination according to claim 4, **characterised in that** the quick-fastener element comprises a sleeve (50) which is mounted in an axially fixed but limitedly rotatable manner in a bore hole (16) of the connection end (10) of the targeting and/or nailing element and the sleeve (50) is provided with a radial actuating pin (58) which projects outward over a radial slot (20) of the connection (12).

6. Combination according to claim 5 in conjunction with claim 2 **characterised in that** the spring (60) is arranged in an interspace between the sleeve (50) and wall of the bore hole (16) in the connection end (10) of the targeting and/or nailing element, the one end of said spring being fastened to the connection end and the other end to the sleeve (50).

7. Combination according to claim 5 and 6, **characterised in that** the sleeve (50) comprises a first radial flange (52) which abuts against a radial abutment surface (32) in the drill hole (16) of the connection end (10) and the sleeve (50) is axially fixed by means of the actuation pin (58) in the opposite direction, wherein the spring (60) lies under axial prestressing against the flange (52) of the sleeve (50) and against the actuation pin or another radial pin bearing the actuation pin (58).

8. Combination according to any one of claims 1 to 7, **characterised in that** the connection end (10) of the targeting and/or nailing instrument comprises a sleeve-shaped end section (28), which engages in an approximate fit in a terminal drill hole section (46) of the connection ends of the nail (40).

9. Combination according to claim 8, **characterised in that** the axially parallel projection (30) of the connection ends (12) of the targeting and/or nailing instrument protrudes axially over the free end of the sleeve-shaped end section (28).

## Revendications

1. Combinaison d'un clou intramédullaire et d'un instrument de visée et/ou de percussion, dans laquelle une extrémité de raccordement (10) de l'instrument de visée et/ou de percussion présente une saillie parallèle à l'axe (30) qui coopère avec un évidement parallèle à l'axe (44) à l'extrémité de raccordement (10) creuse du clou, de telle façon que, du fait de cette coopération, le clou est immobilisé, en étant accolé axialement à l'extrémité de raccordement de l'instrument de visée et/ou de percussion, dans une position de rotation donnée sur l'instrument de visée et/ou de percussion, et des moyens sont prévus pour coupler fermement de façon temporaire le clou avec l'instrument de visée et/ou de percussion, dans laquelle un élément de fermeture rapide est supporté pour tourner à l'extrémité de raccordement (10) de l'instrument de visée et/ou de percussion, lequel élément de fermeture rapide vient en prise dans l'extrémité de raccordement du clou (40) et présente des moyens de couplage et l'extrémité de raccordement du clou (40) présente à l'intérieur des seconds moyens de couplage et les moyens de couplage sont réalisés de telle façon que, dans une première position de rotation de l'élément de fermeture rapide dans laquelle l'élément de fermeture rapide présente une position de rotation relative par rapport à la saillie parallèle à l'axe, l'élément de fermeture rapide peut être inséré dans l'extrémité de raccordement du clou et les moyens de couplage coopèrent dans une seconde position de l'élément de fermeture rapide de telle façon que le clou est maintenu axialement fermement sur l'instrument de visée et/ou de percussion.

2. Combinaison selon la revendication 1, **caractérisée en ce que**, dans la première position de rotation, l'élément de fermeture rapide est précontraint en direction de la seconde position de rotation par un ressort (60), et les moyens de couplage sont réalisés de telle façon qu'ils coopèrent automatiquement, lorsque l'élément de fermeture rapide a été inséré axialement, sur une course donnée, dans l'extrémité de raccordement du clou (40).

3. Combinaison selon la revendication 1 ou 2, **caractérisée en ce que** l'extrémité de raccordement du clou (40) présente à l'intérieur une section filetée (72) et au moins une gorge parallèle à l'axe (70), l'élément de fermeture rapide présente au moins une saillie radiale (64) qui peut être introduite dans la gorge parallèle à l'axe (70) et coopère avec une section de pas de vis de la section filetée (72) lors de la rotation de l'élément de fermeture rapide de la première dans la seconde position de rotation.

4. Combinaison selon la revendication 3, **caractérisée en ce que** trois saillies radiales (64) sont disposées par pas de 120° et de manière décalée axialement d'un pas de vis d'une section filetée (72) et l'intérieur du clou (40 3) présente des rainures parallèles à l'axe, disposées de manière décalée par pas de 120°, la section filetée (72) étant réalisée de telle façon que chaque fois une saillie (64) coopère avec une section de pas de vis de la section filetée (72).

5. Combinaison selon la revendication 4, **caractérisée en ce que** l'élément de fermeture rapide présente une douille (50), qui est supportée de manière ferme axialement, mais pour toutefois tourner de manière limitée dans un perçage (16) de l'extrémité de raccordement (10) de l'instrument de visée et/ou de percussion et la douille (50) est pourvue d'une goupille d'actionnement radiale (58) qui dépasse vers l'extérieur, par une fente radiale (20) de l'extrémité de raccordement (12).

6. Combinaison selon la revendication 5 en liaison avec la revendication 2, **caractérisée en ce que** le ressort (60), dont une extrémité est immobilisée à l'extrémité de raccordement et l'autre extrémité sur la douille (50), est disposé dans un interstice entre la douille (50) et la paroi du perçage (16) dans l'extrémité de raccordement (10) de l'instrument de visée et/ou de percussion.

7. Combinaison selon la revendication 5 et 6, **caractérisée en ce que** la douille (50) présente une première collerette radiale (52), qui est accolée à une surface de butée radiale (32) dans le perçage (16) de l'extrémité de raccordement (10) et la douille (50) est immobilisée axialement dans la direction opposée, au moyen de la goupille d'actionnement (58), le ressort (60) étant accolé, sous précontrainte axiale, à la collerette (52) de la douille (50) et à la goupille d'actionnement ou une autre collerette portant radialement une autre goupille d'actionnement (58).

8. Combinaison selon l'une des revendications 1 à 7, **caractérisée en ce que** l'extrémité de raccordement (10) de l'instrument de visée et/ou de percussion comporte une section d'extrémité (28) en forme de douille, qui vient en prise de manière approximativement ajustée dans une section de perçage (46) du côté extrémité de l'extrémité de raccordement du clou (40).

9. Combinaison selon la revendication 8, **caractérisée en ce que** la saillie parallèle à l'axe (30) de l'extrémité de raccordement (12) de l'instrument de visée et/ou de percussion dépasse axialement par rapport à l'extrémité libre de la section d'extrémité (28) en forme de douille.
